# EUROPÄISCHE PATENTSCHRIFT

(11) **EP 3 884 891 B1**
(45) Veröffentlichungstag und Bekanntmachung des Hinweises auf die Patenterteilung: **18.12.2024**
(21) Anmeldenummer: 21163893.7
(22) Anmeldetag: 22.03.2021
(51) Int. Cl.: A61B 17/70

(54) **CHIRURGISCHE VERBINDUNGSEINRICHTUNG UND CHIRURGISCHES VERBINDUNGSSYSTEM**
SURGICAL CONNECTING DEVICE AND SURGICAL CONNECTION SYSTEM
DISPOSITIF CHIRURGICAL DE LIAISON ET SYSTÈME CHIRURGICAL DE LIAISON

(30) Priorität: 25.03.2020 DE 102020108276
(43) Veröffentlichungstag der Anmeldung: 29.09.2021
(73) Patentinhaber: Aesculap AG, 78532 Tuttlingen (DE)
(72) Erfinder: Schwiesau, Jens, 78532 Tuttlingen (DE); Krüger, Sven, 78647 Trossingen (DE)
(74) Vertreter: Hoeger, Stellrecht & Partner Patentanwälte mbB

(56) Entgegenhaltungen:
- WO-A1-2009/001978
- US-A1- 2010 280 552
- US-A1- 2011 106 166
- US-A1- 2018 098 798
- US-A1- 2018 125 538
- US-B1- 8 998 961

## Beschreibung

Die vorliegende Erfindung betrifft eine chirurgische Verbindungseinrichtung zum Verbinden eines ersten Stabelementes mit einem zweiten Stabelement in einer oder mehr, insbesondere zwei, Relativorientierungen, wobei die Verbindungseinrichtung einen Grundkörper umfasst mit einem ersten Anschlussbereich für das erste Stabelement und einem zweiten Anschlussbereich für das zweite Stabelement, wobei am Grundkörper mindestens drei Aufnahmeöffnungen gebildet sind, wobei an jedem Anschlussbereich mindestens eine der Aufnahmeöffnungen angeordnet ist und mindestens ein Anschlussbereich zwei oder mehr der Aufnahmeöffnungen mit voneinander unterschiedlichen Achsen aufweist, in welche Aufnahmeöffnungen das Stabelement wahlweise einsetzbar ist, und wobei an jedem Anschlussbereich ein Fixierelement zum Fixieren, in einer Fixierrichtung, des jeweiligen Stabelementes am Anschlussbereich angeordnet ist, und wobei eine der Aufnahmeöffnungen an einem ersten Oberflächenabschnitt des Grundkörpers gebildet ist, deren Aufnahmeöffnungsrand eine Durchbrechung aufweist, die sich vom ersten Oberflächenabschnitt bis zu einem benachbarten, im Winkel ausgerichteten zweiten Oberflächenabschnitt des Grundkörpers erstreckt, zum Einführen des Stabelementes in die Aufnahmeöffnung ausgehend vom zweiten Oberflächenabschnitt und durch die Durchbrechung, in einer Einführrichtung quer zur Achse der Aufnahmeöffnung und quer zur Fixierrichtung.

Außerdem betrifft die vorliegende Erfindung ein chirurgisches Verbindungssystem mit einer Verbindungseinrichtung der vorstehend genannten Art und zwei oder mehr Stabelementen, die wahlweise am ersten Anschlussbereich und/oder am zweiten Anschlussbereich fixierbar sind.

Die eingangs genannte Verbindungseinrichtung kommt operativ beispielsweise zum Verbinden von Stabelementen zum Einsatz, die an Verankerungselementen, insbesondere Knochenschrauben, festgelegt sind. Je nach Anwendung sollten die Stabelemente vorzugsweise unterschiedliche Relativorientierungen einnehmen können. Aus diesem Grund ist bei der Verbindungseinrichtung vorgesehen, dass das erste Stabelement am ersten Anschlussbereich und das zweite Stabelement am zweiten Anschlussbereich in einer oder mehreren Relativorientierungen zueinander fixiert werden können. Hierbei können die Stabelemente zum Beispiel jeweils abschnittsweise in einen Aufnahmeraum der Anschlussbereiche eingreifen. Bekannt ist es ferner, dass die Stabelemente unterschiedlich beschaffen sein können. Insbesondere sind Stabelemente unterschiedlichen Durchmessers vorgesehen. In der Praxis zeigt sich, dass eine Vielzahl von unterschiedlichen Verbindungseinrichtungen und Stabelementen vorgehalten werden, die je nach Anwendung zum Einsatz kommen. Dies erschwert die Lagerhaltung. Intraoperativ leidet die Flexibilität. Die Aufbereitung ist erschwert. Lagerung und/oder Aufbereitung erfordern größere Siebkörbe, deren Bestückung unübersichtlich ist. Dies kann einen hohen Kostenaufwand verursachen.

Eine gattungsgemäße Verbindungseinrichtung und ein gattungsgemäßes Verbindungssystem sind beispielsweise in der US 8,998,961 B1 beschrieben. Weitere chirurgische Verbindungssysteme sind in der US 2012/0029571 A1, der US 2008/0125538 A1, der US 2017/0348026 A1, der US 2010/280552 A1, der WO 2009/001978 A1, der US 2018/098798 A1 und der US 2018/125538 A1 offenbart.

Aufgabe der vorliegenden Erfindung ist es, eine gattungsgemäße Verbindungseinrichtung und ein gattungsgemäßes Verbindungssystem bereitzustellen, die bzw. das auf einfachere Weise handhabbar ist.

Diese Aufgabe wird bei einer Verbindungseinrichtung der eingangs genannten Art erfindungsgemäß dadurch gelöst durch den Gegenstand von Anspruch 1. Die erfindungsgemäße Verbindungseinrichtung weist an jedem Anschlussbereich mindestens eine Aufnahmeöffnung auf, wobei mindestens an einem Anschlussbereich zwei oder mehr Aufnahmeöffnungen vorhanden sind, damit die Stabelemente vorzugsweise auch in voneinander unterschiedlichen Relativorientierungen fixiert werden können. "Achse" ist vorliegend insbesondere dahingehend zu verstehen, dass eine jeweilige Aufnahmeöffnung eine Orientierung (Ausrichtung, speziell weg vom Grundkörper, und Position) des in der Aufnahme bestimmungsgemäß angeordneten Stabelementes definiert. Erfindungsgemäß ist an einem ersten Oberflächenabschnitt eine Aufnahmeöffnung vorgesehen, deren Aufnahmeöffnungsrand eine Durchbrechung aufweist. Dies gibt die Möglichkeit, das zu fixierende Stabelement ausgehend von einem zweiten Oberflächenabschnitt durch die Durchbrechung hindurch in die Aufnahmeöffnung einzuführen. Die Einführrichtung ist quer zur Achse und quer zur Fixierrichtung des Fixierelementes ausgerichtet. Auf diese Weise ist die Möglichkeit gegeben, das Stabelement nicht nur entlang der Achse, gewissermaßen "axial" in die Aufnahmeöffnung einzuführen, sondern quer dazu, seitlich durch die Durchbrechung hindurch. Je nach zur Verfügung stehendem Raum, Anwendung und/oder Operationstechnik kann sich der Operateur für eine der Einführrichtungen entscheiden, wodurch die Verbindungseinrichtung vielseitiger einsetzbar und einfacher handhabbar ist.

Am jeweiligen Anschlussbereich kann zum Beispiel ein Aufnahmeraum angeordnet sein, in den das Stabelement eingreift und in dem es mittels des Fixierelementes fixierbar ist.

Vorteilhaft kann es insbesondere sein, dass das Stabelement durch die Durchbrechung quer zur Fixierrichtung in die Aufnahmeöffnung eingeführt werden kann. Dies bietet vorzugsweise die Möglichkeit, das Fixierelement am Grundkörper vorzufixieren, das Stabelement einzuführen und anschließend mit dem Fixierelement zu fixieren. Auch dies begünstigt die verbesserte Handhabung der erfindungsgemäßen Verbindungseinrichtung.

"Quer" kann vorliegend insbesondere "senkrecht" bedeuten und einschließen.

Die Aufnahmeöffnung am ersten Oberflächenabschnitt wird nachfolgend auch als erste Aufnahmeöffnung bezeichnet.

Günstigerweise ist eine Öffnungsweite der Durchbrechung kleiner als die Öffnungsweite der Aufnahmeöffnung am ersten Oberflächenabschnitt. Dies verringert vorzugsweise die Wahrscheinlichkeit, dass das in die Aufnahmeöffnung eingeführte Stabelement unbeabsichtigterweise wieder aus der Aufnahmeöffnung durch die Durchbrechung gelangt, und verbessert die Handhabung der Verbindungseinrichtung.

Erfindungsgemäß bildet oder umfasst die Aufnahmeöffnung bezüglich der Durchbrechung eine Vertiefung, in die das Stabelement eingeführt und darin mittels des Fixierelementes fixiert werden kann. Nach dem Durchgang durch die Durchbrechung kann das Stabelement zum Beispiel in die Vertiefung eingelegt und darin fixiert werden.

Der Grundkörper kann in einer Draufsicht längs der Achse abschnittsweise zum Beispiel im Wesentlichen C-förmig ausgestaltet sein, wobei die Durchbrechung am Aufnahmeöffnungsrand zwischen den beiden Schenkeln des C gebildet ist. Derjenige Schenkel, im Bereich dessen das Fixierelement angeordnet ist, kann insbesondere länger ausgestreckt sein als der andere Schenkel des C.

Beispielsweise ist der Schenkel des C, an dessen Bereich die vorstehend genannte Vertiefung angeordnet ist, kürzer ausgestaltet als der andere Schenkel des C. Der verkürzt ausgestaltete Schenkel ist beispielsweise derjenige Schenkel, der dem längeren Schenkel gegenüberliegt, im Bereich dessen das Fixierelement angeordnet ist.

Am zweiten Oberflächenabschnitt ist eine zweite Aufnahmeöffnung gebildet ist mit einem Aufnahmeöffnungsrand, der die Durchbrechung aufweist, zum Einführen des Stabelementes in die zweite Aufnahmeöffnung ausgehend vom ersten Oberflächenabschnitt und durch die Durchbrechung hindurch, quer zur Achse der zweiten Aufnahmeöffnung. Angeordnet in der zweiten Aufnahmeöffnung weist das Stabelement im Verhältnis zu einer Anordnung in der ersten Aufnahmeöffnung eine unterschiedliche Orientierung auf, im Hinblick auf verschiedenartige Relativorientierungen zu dem anderen Stabelement. Durch die Durchbrechung hindurch kann das Stabelement nicht nur in die erste Aufnahmeöffnung eingeführt werden, sondern, in unterschiedlicher Orientierung, in die zweite Aufnahmeöffnung. Die diesbezügliche Einführrichtung ist quer zur Achse der zweiten Aufnahmeöffnung und insbesondere quer zur Fixierrichtung ausgerichtet.

Vorteilhaft ist es, wenn der Aufnahmeöffnungsrand der zweiten Aufnahmeöffnung eine weitere Durchbrechung aufweist, zum Einführen des Stabelementes in die zweite Aufnahmeöffnung ausgehend von einem dritten Oberflächenabschnitt des Grundkörpers, der dem ersten Oberflächenabschnitt abgewandt ist, und durch die weitere Durchbrechung hindurch, quer zur Achse der zweiten Aufnahmeöffnung. Dies erlaubt es, das Stabelement auf andersartige Weise in die zweite Aufnahmeöffnung einzuführen. Die diesbezügliche Einführrichtung ist quer zu deren Achse und insbesondere entgegengesetzt der Einführrichtung in die zweite Aufnahmeöffnung ausgehend vom ersten Oberflächenabschnitt ausgerichtet. Insbesondere kann die weitere Einführrichtung quer zur Fixierrichtung ausgerichtet sein. Durch die weitere Durchbrechung wird die Vielseitigkeit der Verbindungseinrichtung gesteigert und dadurch die Handhabbarkeit verbessert.

Günstigerweise ist an einem dritten Oberflächenabschnitt, der dem ersten Oberflächenabschnitt abgewandt ist, eine dritte Aufnahmeöffnung gebildet. Das Stabelement kann in die dritte Aufnahmeöffnung eingeführt werden und darin mittels des Fixierelementes fixiert werden.

Von Vorteil ist es, wenn ein Aufnahmeöffnungsrand der dritten Aufnahmeöffnung die vorstehend genannte weitere Durchbrechung aufweist, zum Einführen des Stabelementes in die dritte Aufnahmeöffnung ausgehend vom zweiten Oberflächenabschnitt und durch die weitere Durchbrechung hindurch, quer zur Achse. Dies bietet die Möglichkeit, das Stabelement in die dritte Aufnahmeöffnung nicht nur in Erstreckungsrichtung des Stabelementes axial, sondern durch die weitere Durchbrechung ausgehend vom zweiten Oberflächenabschnitt einzuführen. Dies erhöht die Vielseitigkeit der Verbindungseinrichtung und verbessert deren Handhabbarkeit. Insbesondere ist die diesbezügliche Einführrichtung quer zur Fixierrichtung ausgerichtet.

Nach dem Gesagten ist vorzugsweise vorgesehen, dass der Aufnahmeöffnungsrand der zweiten Aufnahmeöffnung zwei Durchbrechungen aufweist. Eine erste Durchbrechung ist derart gebildet, dass sie zugleich eine Durchbrechung des Aufnahmeöffnungsrandes der ersten Aufnahmeöffnung ist. Eine zweite Durchbrechung ist derart gebildet, dass sie zugleich eine Durchbrechung des Aufnahmeöffnungsrandes der dritten Aufnahmeöffnung ist.

Insbesondere kann der Grundkörper Kantenabschnitte aufweisen, an denen Durchbrechungen gebildet sind. Durch eine der Durchbrechungen kann ein Stabelement je nach gewählter Einführrichtung und Orientierung des Stabelementes in die erste Aufnahmeöffnung oder in die zweite Aufnahmeöffnung eingeführt werden. Durch die zweite Durchbrechung kann ein Stabelement je nach gewählter Einführrichtung und Orientierung des Stabelementes in die zweite Aufnahmeöffnung oder in die dritte Aufnahmeöffnung eingeführt werden.

Vorgesehen sein kann, dass die dritte Aufnahmeöffnung koaxial zur Aufnahmeöffnung am ersten Oberflächenabschnitt ausgerichtet ist. Dies gibt beispielsweise die Möglichkeit, ein Stabelement durch die erste und die dritte Aufnahmeöffnung hindurch zu führen.

Wenn der Aufnahmeöffnungsrand der ersten Aufnahmeöffnung und der Aufnahmeöffnungsrand der dritten Aufnahmeöffnung eine jeweilige Durchbrechung zum Einführen eines Stabelementes ausgehend vom zweiten Oberflächenabschnitt aufweisen, besteht vorzugsweise insbesondere die Möglichkeit, ein Stabelement durch diese beiden Durchbrechungen hindurch ausgehend vom zweiten Oberflächenabschnitt quer zur jeweiligen Achse und insbesondere quer zur Fixierrichtung in die erste und die dritte Aufnahmeöffnung einzuführen.

Die dritte Aufnahmeöffnung weist vorzugsweise eine zur Aufnahmeöffnung am ersten Oberflächenabschnitt identische Form auf. Die Aufnahmeöffnungen können insbesondere kongruent sein.

Vorgesehen sein kann, dass eine Öffnungsweite der Durchbrechung (zum Einführen des Stabelementes ausgehend von dem ersten Oberflächenabschnitt in die zweite Aufnahmeöffnung) kleiner ist als die Öffnungsweite der zweiten Aufnahmeöffnung.

Alternativ oder ergänzend kann vorgesehen sein, dass eine Öffnungsweite der weiteren Durchbrechung kleiner ist als die Öffnungsweite der zweiten Aufnahmeöffnung.

Alternativ oder ergänzend kann vorgesehen sein, dass eine Öffnungsweite der weiteren Durchbrechung kleiner ist als die Öffnungsweite der dritten Aufnahmeöffnung.

Durch die im Verhältnis zur jeweiligen Aufnahmeöffnung geringere Öffnungsweite der jeweiligen Durchbrechung ist die Wahrscheinlichkeit verringert, dass das Stabelement seitlich aus der Aufnahmeöffnung gelangt. Die Handhabung der Verbindungseinrichtung ist dadurch verbessert.

Erfindungsgemäß ist vorgesehen, dass die zweite Aufnahmeöffnung und optional die dritte Aufnahmeöffnung eine Vertiefung bezüglich der erstgenannten Durchbrechung oder der weiteren Durchbrechung bildet oder umfasst, in welcher das Stabelement mittels des Fixierelementes fixiert werden kann. Nach dem Durchgang durch die Durchbrechung oder die weitere Durchbrechung kann das Stabelement zum Beispiel in die Vertiefung der zweiten Aufnahmeöffnung eingelegt und darin fixiert werden. In entsprechender Weise kann das Stabelement nach dem Durchgang durch die weitere Durchbrechung zum Beispiel in die dritte Aufnahmeöffnung eingelegt und darin fixiert werden.

Vorgesehen sein kann, dass die zweite Aufnahmeöffnung eine von der Form der Aufnahmeöffnung am ersten Oberflächenabschnitt (erste Aufnahmeöffnung) und/oder von der Form der dritten Aufnahmeöffnung abweichende Form aufweist.

Günstigerweise ist vorgesehen, dass die zweite Aufnahmeöffnung eine Achse definiert, die senkrecht zu der Achse der Aufnahmeöffnung am ersten Oberflächenabschnitt (erste Aufnahmeöffnung) und/oder zu der Achse der dritten Aufnahmeöffnung ausgerichtet ist. Die Achsen können sich insbesondere schneiden.

Bei einer bevorzugten Ausführungsform ist vorgesehen, dass der zweite Oberflächenabschnitt senkrecht zum ersten Oberflächenabschnitt und/oder senkrecht zum dritten Oberflächenabschnitt ausgerichtet ist.

Alternativ oder ergänzend kann vorgesehen sein, dass der erste und der dritte Oberflächenabschnitt parallel zueinander ausgerichtet sind.

Das Fixierelement erlaubt beispielsweise eine kraft- oder formschlüssige Fixierung des Stabelementes und kann zum Beispiel kraft- und/oder formschlüssig am Anschlussbereich fixiert sein.

Am jeweiligen Anschlussbereich ist für das jeweilige Fixierelement beispielsweise eine Fixierelementaufnahme gebildet. Vorzugsweise kann insbesondere vorgesehen sein, dass ein Rand mindestens einer Fixierelementaufnahme in sich geschlossen und/oder frei von einer Durchbrechung ist. Auf diese Weise kann ein zuverlässiger Sitz des Fixierelementes in der Fixierelementaufnahme gesichert werden.

Insbesondere ist vorzugsweise nicht vorgesehen, dass ein Stabelement durch eine Durchbrechung eines Randes der Fixierelementaufnahme hindurch in eine der Aufnahmeöffnungen einführbar ist.

Bei einer bevorzugten Ausführungsform ist das Fixierelement beispielsweise ein Schraubelement, das in der Fixierrichtung in eine Schraubaufnahme am Grundkörper einschraubbar ist, wobei das Stabelement mittels des Schraubelementes klemmend am Anschlussbereich fixierbar ist. Die Schraubaufnahme bildet die vorstehend genannte Fixierelementaufnahme und weist dabei insbesondere ein Gewinde auf, das mit einem Gewinde des Schraubelementes in Eingriff bringbar ist.

Vorgesehen sein kann insbesondere, dass nur an einem Anschlussbereich eine Aufnahmeöffnung mit einem eine Durchbrechung aufweisenden Aufnahmeöffnungsrand wie vorstehend beschrieben vorgesehen ist.

Günstig ist es, wenn am ersten Anschlussbereich und am zweiten Anschlussbereich zwei an einander abgewandten Oberflächenabschnitten gebildete Aufnahmeöffnungen gebildet sind, wobei die Stabelemente in einander entgegengesetzten Richtungen in die Aufnahmeöffnungen einführbar sind. Dies gibt die Möglichkeit, die Stabelemente über die Verbindungseinrichtung axial miteinander zu verbinden, wobei an jedem der beiden Anschlussbereiche eines der Stabelemente festgelegt ist. Die Aufnahmeöffnungen können koaxial zueinander ausgerichtet sein und miteinander fluchtende Achsen definieren. Bei Stabelementen gleichen Durchmessers können Achsen der Stabelemente miteinander fluchten. Bei Stabelementen unterschiedlichen Durchmessers können die Achsen der Stabelemente parallel zueinander ausgerichtet sein.

Vorzugsweise umfasst der Grundkörper ein in einer der Einführrichtungen wirksames Anschlagelement für mindestens eines der Stabelemente. Das Anschlagelement am Grundkörper kann insbesondere dazu dienen, dass zumindest eines der Stabelemente eine definierte Relativposition zum Grundkörper einnimmt. Dies verbessert die Handhabbarkeit der Verbindungseinrichtung.

Das Anschlagelement begrenzt beispielsweise die vorstehend genannten Aufnahmeräume der Anschlussbereiche.

Das Anschlagelement kann bei einer bevorzugten Ausführungsform als Zwischenwand des Grundkörpers ausgebildet sein. Insbesondere kann ein Anschlagelement, insbesondere die Zwischenwand, für beide der Stabelemente wirksam sein. Die Zwischenwand kann vollständig geschlossen und frei von einer Öffnung sein.

Die Anschlussbereiche sind bei einer bevorzugten Ausführungsform der Erfindung starr miteinander verbunden. Hierunter kann vorliegend insbesondere verstanden werden, dass die Anschlussbereiche so fest miteinander verbunden sind, dass sie nicht beweglich aneinander festlegbar sind. Vielmehr kann bei der bevorzugten Ausführungsform eine unbewegliche Verbindung der Anschlussbereiche vorgesehen sein.

Vorteilhafterweise ist am ersten Anschlussbereich mindestens eine Aufnahmeöffnung gebildet und am zweiten Anschlussbereich mindestens eine weitere Aufnahmeöffnung gebildet, die Achsen definieren, die quer und insbesondere senkrecht zueinander ausgerichtet sind. Dies gibt die Möglichkeit, die Stabelemente im Winkel relativ zueinander mittels der Verbindungseinrichtung zu verbinden. Hierbei kann insbesondere eine rechtwinklige Verbindung vorgesehen sein, wobei die Stabelemente beispielsweise in L-Form zueinander ausgerichtet sind.

Bevorzugt ist am ersten Anschlussbereich mindestens eine Aufnahmeöffnung gebildet und am zweiten Anschlussbereich mindestens eine weitere Aufnahmeöffnung gebildet, die Achsen definieren, die parallel zueinander ausgerichtet sind. Dies gibt die Möglichkeit, zwei parallel zueinander orientierte Stabelemente mittels der Verbindungseinrichtung zu verbinden.

Bei den beiden zuletzt erwähnten vorteilhaften Ausführungsformen der Erfindung ist es günstig, wenn an zumindest einem der Anschlussbereiche zwei koaxial zueinander ausgerichtete Aufnahmeöffnungen gebildet sind, und wenn das Stabelement durch die beiden Aufnahmeöffnungen hindurch führbar ist. Beispielsweise sind diese Aufnahmeöffnungen an zwei einander abgewandten Seiten des entsprechenden Anschlussbereiches gebildet, und das Stabelement kann durch die Aufnahmeöffnungen und den Grundkörper hindurch geführt sein. Die Aufnahmeöffnungen können insbesondere von identischer Form und/oder kongruent sein.

Günstig ist es, wenn zumindest einer der Anschlussbereiche zum Fixieren von Stabelementen unterschiedlichen Durchmessers ausgebildet ist. Dies kann beispielsweise wie nachfolgend erläutert durch die Gestalt der Aufnahmeöffnungen begünstigt werden.

Bei einer bevorzugten Ausführungsform der Verbindungseinrichtung ist zumindest eine Aufnahmeöffnung mit einem kreisrunden oder mit einem unrunden Querschnitt vorgesehen.

Günstig ist es, wenn zumindest eine Aufnahmeöffnung vorgesehen ist, deren Aufnahmeöffnungsrand segmentweise entlang eines Kreisbogens mit einem ersten Radius und segmentweise entlang eines Kreisbogens mit einem zweiten Radius verläuft, der zum ersten Radius unterschiedlich ist und insbesondere kleiner als der erste Radius ist. Durch die unterschiedlichen Radien ist die Aufnahmeöffnung besser zum Fixieren von Stabelementen unterschiedlicher Radien geeignet. Beispielsweise kann ein Stabelement mit kleinerem Radius an den Kreisbogen mit zweitem Radius (wenn dies der kleinere Radius ist) angelegt werden. Ein Stabelement mit größerem Radius kann an den Kreisbogen mit erstem Radius angelegt werden (wenn dies der größere Radius ist).

Als günstig kann es sich erweisen, wenn zumindest eine Aufnahmeöffnung vorgesehen ist, deren Aufnahmeöffnungsrand zwei planare und V-förmig zueinander ausgerichtete Segmente aufweist. Durch die Segmente besteht die Möglichkeit, eine Mehrzahl von Stabelementen unterschiedlicher Radien gleichermaßen am Aufnahmeöffnungsrand zu adaptieren.

Zwischen den planaren Segmenten, die V-förmig zueinander ausgerichtet sind, weist der Aufnahmeöffnungsrand vorzugsweise ein Segment mit Radius auf.

Die eingangs genannte Aufgabe wird durch ein erfindungsgemäßes chirurgisches Verbindungssystem gelöst, das mindestens eine Verbindungseinrichtung der vorstehend genannten Art und zwei oder mehr Stabelemente umfasst, die wahlweise am ersten Anschlussbereich und/oder am zweiten Anschlussbereich fixierbar sind.

Die bereits im Zusammenhang mit der Erläuterung der erfindungsgemäßen Verbindungseinrichtung erwähnten Vorteile können mit dem Verbindungssystem ebenfalls erzielt werden. Zur Vermeidung von Wiederholungen kann auf die voranstehenden Ausführungen verwiesen werden.

Vorteilhafte Ausführungsformen des erfindungsgemäßen Verbindungssystems ergeben sich durch vorteilhafte Ausführungsformen der erfindungsgemäßen Verbindungseinrichtung. Auch diesbezüglich wird auf die voranstehenden Ausführungen verwiesen.

Die Stabelemente können identisch ausgestaltet sein. Alternativ oder ergänzend kann vorgesehen sein, dass zumindest zwei unterschiedlich ausgestaltete Stabelemente vorgesehen sind. Beispielsweise sind zumindest zwei Stabelemente mit unterschiedlichen Radien vorgesehen.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine erste perspektivische Darstellung eines erfindungsgemäßen Verbindungssystems, umfassend eine erfindungsgemäße Verbindungseinrichtung und zwei Stabelemente in einer ersten Relativorientierung;
- Figur 2:: eine perspektivische Darstellung eines Grundkörpers der Verbindungseinrichtung aus Figur 1;
- Figur 3:: eine Seitenansicht des Grundkörpers;
- Figur 4:: eine weitere perspektivische Darstellung des Grundkörpers;
- Figur 5:: eine zweite perspektivische Darstellung des Verbindungssystems aus Figur 1, wobei die Stabelemente eine zweite Relativorientierung einnehmen;
- Figur 6:: eine dritte perspektivische Darstellung des Verbindungssystems aus Figur 1, wobei die Stabelemente eine dritte Relativorientierung einnehmen;
- Figur 7:: eine Schnittansicht längs der Linie 7-7 in Figur 3;
- Figuren 8 und 9:: eine vergrößerte Darstellung eines Teilbereichs des Verbindungssystems, in der eine Aufnahmeöffnung mit einem jeweiligen darin angeordneten Stabelement dargestellt ist, wobei die Stabelemente unterschiedliche Radien aufweisen; und
- Figuren 10 und 11:: eine Darstellung ähnlich der Figuren 8 und 9 bei einer weiteren bevorzugten Ausführungsform der Erfindung, wobei in einer Aufnahmeöffnung Stabelemente mit unterschiedlichen Radien angeordnet sind.

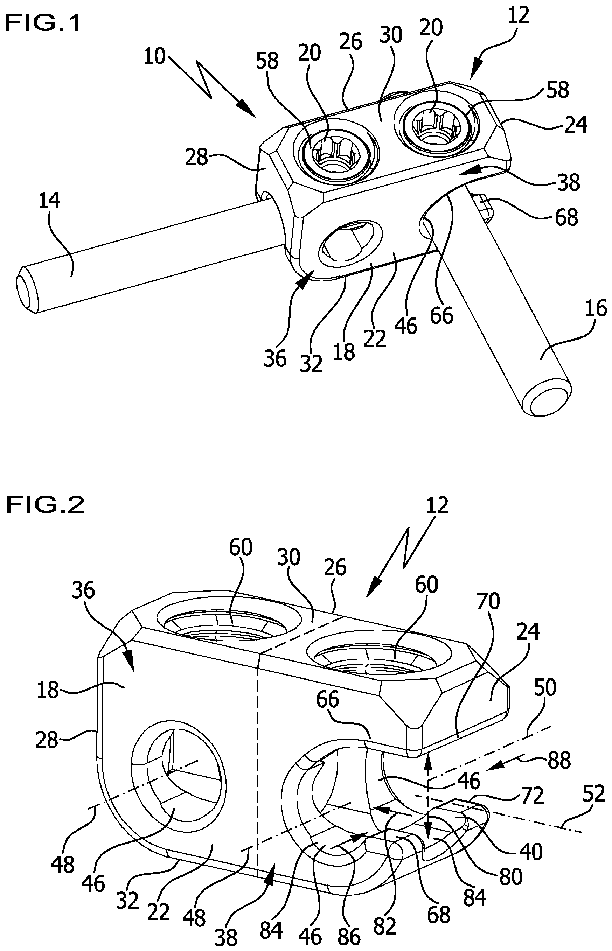

Die Figuren 1, 5 und 6 zeigen jeweils eine perspektivische Darstellung einer bevorzugten Ausführungsform eines erfindungsgemäßen Verbindungssystems 10. Das Verbindungssystem 10 umfasst eine bevorzugte Ausführungsform einer erfindungsgemäßen vielseitigen und universell einsetzbaren Verbindungseinrichtung 12 und mindestens zwei Stabelemente 14, 16.

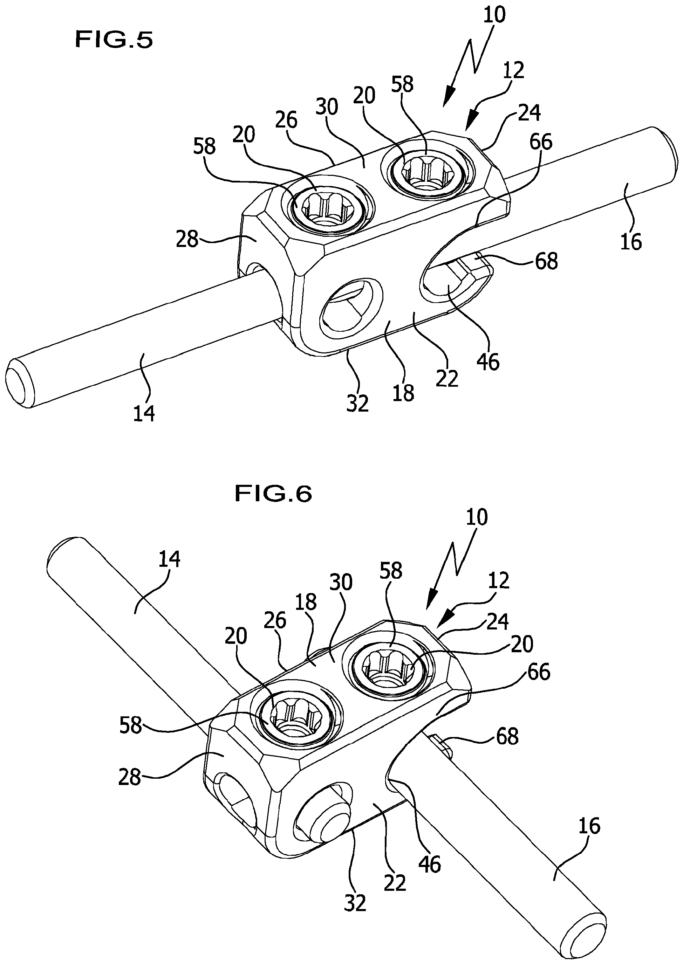

Wie aus den nachfolgenden Erläuterungen hervorgeht und unmittelbar aus den Figuren 1, 5 und 6 ersichtlich ist, können die Stabelemente 14, 16 vorzugsweise in unterschiedlichen Relativorientierungen zueinander mittels der Verbindungseinrichtung 12 verbunden werden. Die unterschiedlichen Relativanordnungen umfassen insbesondere eine L-förmige Relativorientierung (Figur 1), eine axiale Verbindung (Figur 5) und eine parallele Relativorientierung der Stabelemente 14, 16 (Figur 6).

Die Verbindungseinrichtung 12 umfasst einen Grundkörper 18 und zwei Fixierelemente 20. Der Grundkörper 18 ist vorliegend einstückig ausgestaltet.

Im vorliegenden Fall ist der Grundkörper 18 von seiner Grundform ungefähr quaderförmig oder an eine Quaderform angelehnt. Der Grundkörper 18 weist einen ersten Oberflächenabschnitt 22 und an diesen angrenzenden zweiten Oberflächenabschnitt 24 auf, der quer und insbesondere senkrecht zum ersten Oberflächenabschnitt 22 ausgerichtet ist.

Der Grundkörper 18 weist einen dritten Oberflächenabschnitt 26 auf, der an den zweiten Oberflächenabschnitt 24 angrenzt und quer und insbesondere senkrecht zu diesem ausgerichtet ist. Die Oberflächenabschnitte 22 und 26 sind an einander abgewandten Seiten des Grundkörpers 18 angeordnet.

Ein vierter Oberflächenabschnitt 28 verbindet die Oberflächenabschnitte 22, 26 miteinander und ist zu diesen quer und insbesondere senkrecht ausgerichtet. Die Oberflächenabschnitte 24, 28 sind an einander abgewandten Seiten des Grundkörpers 18 angeordnet.

Des Weiteren sind ein fünfter Oberflächenabschnitt 30 und ein sechster Oberflächenabschnitt 32 vorgesehen, an einander abgewandten Seiten des Grundkörpers 18. Die Oberflächenabschnitte 30, 32 grenzen jeweils an die anderen Oberflächenabschnitte 22 bis 28.

Es versteht sich, dass im Bereich der Übergänge einander benachbarter Oberflächenabschnitte 22 bis 32 und/oder an Eckbereichen des Grundkörpers 18 Abschrägungen oder Fasen vorhanden sein können.

Der Grundkörper 18 ist insgesamt spiegelsymmetrisch bezüglich einer Mittellängsebene 34. Die Mittellängsebene 34 ist mittig zwischen den Oberflächenabschnitten 22 und 26 angeordnet und verläuft parallel zu den von diesen beiden Oberflächenabschnitten definierten Ebenen.

Der Grundkörper 18 umfasst einen ersten Anschlussbereich 36 und einen zweiten Anschlussbereich 38. Am ersten Anschlussbereich 36 kann eines der Stabelemente 14, 16 festgelegt werden. Die Zeichnung zeigt dies vorliegend am Beispiel des Stabelementes 14. Am zweiten Anschlussbereich 38 kann das jeweils andere Stabelement 14, 16 festgelegt werden. In der Zeichnung ist dies am Beispiel des Stabelementes 16 gezeigt.

Die Anschlussbereiche 36, 38 sind voneinander beispielsweise durch eine Querebene 40 des Grundkörpers 18 getrennt. Die Querebene 40 ist senkrecht zur Mittellängsebene 34 ausgerichtet und ungefähr mittig zwischen den Oberflächenabschnitten 24 und 28 angeordnet.

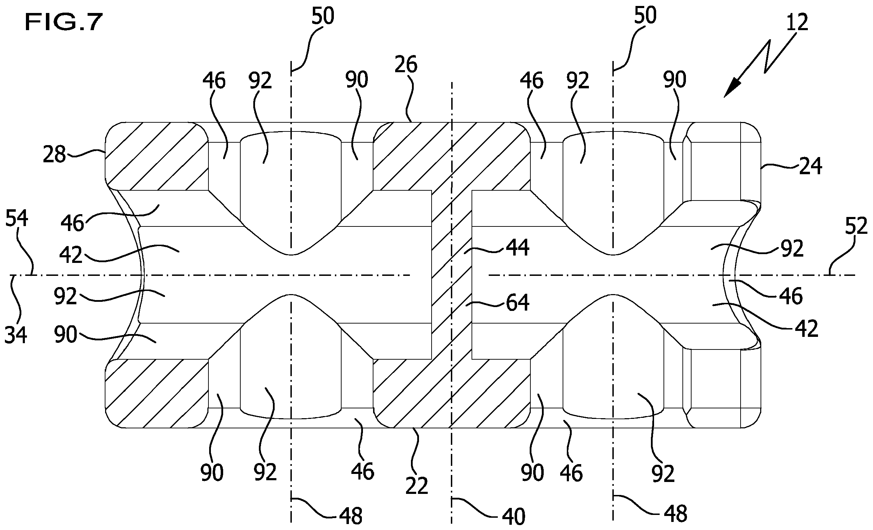

Wie insbesondere aus Figur 7 hervorgeht, ist an jedem Anschlussbereich 36, 38 im Grundkörper 18 ein Hohlraum 42 gebildet. Die Hohlräume 42 sind voneinander durch eine Zwischenwand 44 getrennt, durch die hindurch die Querebene 40 verläuft.

Der Hohlraum 42 ist vorliegend ein am jeweiligen Anschlussbereich 36, 38 gebildeter Aufnahmeraum. Ein jeweiliges Stabelement 14, 16 kann in den Hohlraum 42 eingreifen. Der Hohlraum 42 ist ein Aufnahmeraum des Anschlussbereiches 36 oder 38.

Zum Einführen der Stabelemente 14, 16 in den jeweiligen Hohlraum 42 sind am Grundkörper 18 mehrere Aufnahmeöffnungen an den Oberflächenabschnitten 22 bis 28 gebildet. Die Aufnahmeöffnungen sind jeweils mit dem Bezugszeichen 46 bezeichnet. Über eine jeweilige Aufnahmeöffnung 46 kann ein Stabelement 14, 16 in einen der Anschlussbereiche 36, 38 in den Hohlraum 42 eingreifend eingeführt oder durch diesen hindurch geführt werden. Je nachdem, welche Orientierung die Stabelemente 14, 16 in den Aufnahmeöffnungen 46 einnehmen, können auf diese Weise die unterschiedlichen, in den Figuren 1, 5 und 6 gezeigten Relativorientierungen der Stabelemente 14, 16 umgesetzt werden.

Eine jeweilige Aufnahmeöffnung 46 weist eine Achse auf. Die Achse kann insbesondere als diejenige Richtung angesehen werden, in der ein Stabelement 14, 16 in einer jeweiligen Aufnahmeöffnung 46 aus dem Grundkörper 18 herausragt. Hierbei handelt es sich beispielsweise um eine Achse der jeweiligen Aufnahmeöffnung 46.

Die Aufnahmeöffnungen 46 am ersten Oberflächenabschnitt 22 weisen eine Achse 48 auf. Die Aufnahmeöffnungen 46 am dritten Oberflächenabschnitt 26 weisen eine Achse 50 auf, die der Achse 48 entgegengesetzt ist.

Die Aufnahmeöffnung 46 am zweiten Oberflächenabschnitt 24 weist eine Achse 52 auf, die quer und insbesondere senkrecht zu den Achsen 48, 50 ausgerichtet ist.

Die Aufnahmeöffnung 46 am vierten Oberflächenabschnitt 28 weist eine Achse 54 auf, die entgegen der Achse 52 ausgerichtet und quer und insbesondere senkrecht zu den Achsen 48, 50 ausgerichtet ist.

Um die Stabelemente 14, 16 an den Anschlussbereichen 36 bzw. 38 festzulegen, umfasst die Verbindungseinrichtung 12 die Fixierelemente 20. Vorliegend sind zwei Fixierelemente 20 vorgesehen, die jeweils als Schraubelement 58 ausgestaltet und in einer Schraubaufnahme 60 an den Anschlussbereichen 36, 38 angeordnet sind. Die Schraubaufnahmen 60 sind im fünften Oberflächenabschnitt 30 gebildet.

Die Schraubaufnahme 60 ist eine Fixierelementaufnahme für das Fixierelement 20. Ein Rand der jeweiligen Schraubaufnahme 60 ist in sich geschlossen und frei von einer Durchbrechung. Insbesondere kann keines der Stabelemente 14, 16 durch eine derartige Durchbrechung des Randes der Schraubaufnahme 60 hindurch in eine der Aufnahmeöffnungen 46 eingeführt werden.

Die Schraubelemente 58 können in einer Fixierrichtung 62, die auf den Hohlraum 42 und den sechsten Oberflächenabschnitt 32 gerichtet ist, eingeschraubt werden. Je nachdem, wie dick das Stabelement 14, 16 ist, greifen die Schraubelemente 58 mehr oder weniger weit in den Hohlraum 42 ein. Die Stabelemente 14, 16 werden mittels der Schraubelemente 58 klemmend am Grundkörper 18 fixiert.

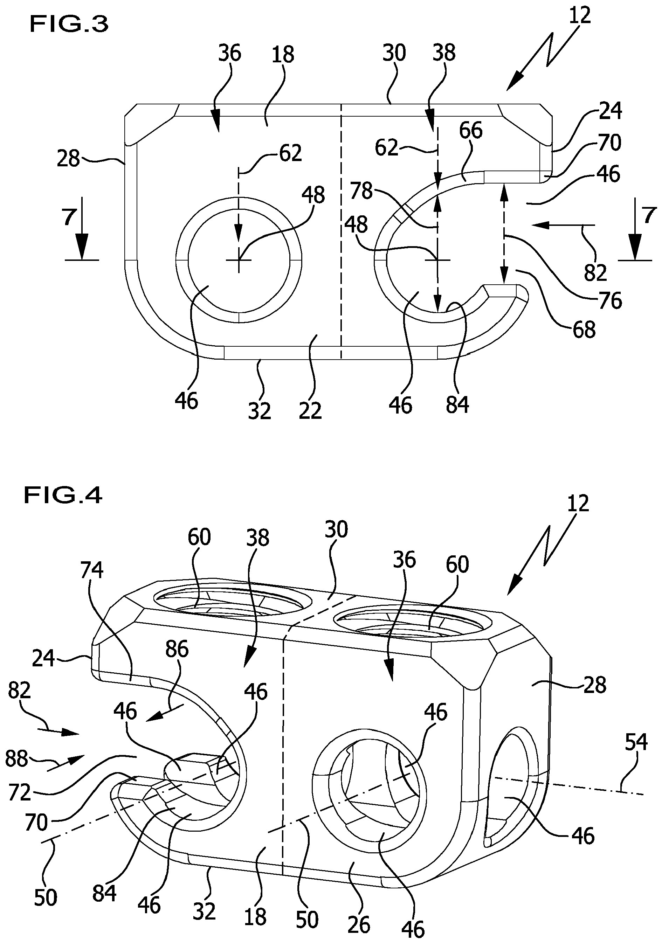

Der erste Anschlussbereich 36 umfasst an den Oberflächenabschnitten 22, 26 und 28 jeweils eine Aufnahmeöffnung 46. Die Aufnahmeöffnungen 46 der Oberflächenabschnitte 22 und 26 sind koaxial zueinander ausgerichtet (Figur 3). Darüber hinaus sind die Aufnahmeöffnungen 46 der Oberflächenabschnitte 22 und 26 von identischer Form und kongruent zueinander.

Die Achsen 48 und 50 fluchten miteinander, sind aber hinsichtlich der Orientierung weg vom Grundkörper 18 einander entgegengesetzt. Die Achse 54 ist quer und insbesondere senkrecht zu den Achsen 48, 50 ausgerichtet.

Am ersten Anschlussbereich 36 kann das Stabelement 14 folgendermaßen angeordnet werden:
Erstens besteht die Möglichkeit, das Stabelement 14 in der Achse 54 und durch die Aufnahmeöffnung 46 am vierten Oberflächenabschnitt 28 auszurichten (Figuren 1 und 5). Die Zwischenwand 44 dient vorzugsweise als Anschlagelement 46 für das Stabelement 14.

Zweitens besteht die Möglichkeit, das Stabelement 14 nur durch die Aufnahmeöffnung 46 am ersten Oberflächenabschnitt 22 oder nur durch die Aufnahmeöffnung 46 am dritten Oberflächenabschnitt 26 in der Achse 48 bzw. 50 auszurichten. Dies ist in der Zeichnung nicht dargestellt.

Drittens besteht die Möglichkeit, das Stabelement 14 durch beide Aufnahmeöffnungen 46 der Oberflächenabschnitte 22, 26 durch den Grundkörper 18 hindurch zu führen. Dies ist in Figur 6 dargestellt, wobei in diesem Fall das Stabelement 16 längs der Achse 50 vom Grundkörper 18 absteht.

Auch am zweiten Anschlussbereich 38 sind, wie erwähnt, Aufnahmeöffnungen 46 vorhanden, zum Eingreifen des Stabelementes 16 in den Hohlraum 42 oder zum Hindurchführen durch den Grundkörper 18.

Wie insbesondere aus den Figuren 2 und 3 hervorgeht, ist am ersten Oberflächenabschnitt 22 eine Aufnahmeöffnung 46 gebildet, die einen Aufnahmeöffnungsrand 66 aufweist. Der Aufnahmeöffnungsrand 66 weist eine Durchbrechung 68 in Richtung des zweiten Oberflächenabschnittes 24 auf.

Am zweiten Oberflächenabschnitt 22 ist eine Aufnahmeöffnung 46 gebildet, die einen Aufnahmeöffnungsrand 70 aufweist. Der Aufnahmeöffnungsrand 70 weist zwei Durchbrechungen auf. Zu diesen zählen die bereits erwähnte Durchbrechung 68 in Richtung des ersten Oberflächenabschnittes 22. Darüber hinaus ist der Durchbrechung 68 gegenüberliegend eine weitere Durchbrechung 72 vorgesehen, die sich bis zum dritten Oberflächenabschnitt 26 erstreckt.

Wie insbesondere aus Figur 4 hervorgeht, ist am dritten Oberflächenabschnitt 26 eine Aufnahmeöffnung 46 gebildet, die einen Aufnahmeöffnungsrand 74 aufweist. Der Aufnahmeöffnungsrand 74 weist eine Durchbrechung auf. Hierbei handelt es sich um die Durchbrechung 72, die sich bis zum zweiten Oberflächenabschnitt 24 erstreckt.

Die Aufnahmeöffnungen 46 an den Oberflächenabschnitten 22 und 26 sind vorliegend von identischer Form und koaxial zueinander ausgerichtet sowie kongruent zueinander.

Die vorstehend erläuterte Konfiguration kann im vorliegenden Beispiel des Grundkörpers 18 dahingehend formuliert werden, dass der Grundkörper 18 am zweiten Anschlussbereich 38 zwei Durchbrechungen aufweist. Die erste Durchbrechung 68 ist eine Durchbrechung der Aufnahmeöffnungsränder 66 und 70 und im Übergang zwischen den Oberflächenabschnitten 22 und 24 gebildet. Die weitere Durchbrechung 72 ist eine Durchbrechung der Aufnahmeöffnungsränder 70 und 74 und im Übergang der Oberflächenabschnitte 24 und 26 gebildet.

Wie insbesondere aus den Figuren 2 bis 4 hervorgeht, sind die Durchbrechungen 68, 72 vorliegend mit einer identischen Öffnungsweite 76 versehen. Die Öffnungsweite 76 kann bei einer bevorzugten Ausführungsform kleiner sein als die Öffnungsweite 78 der Aufnahmeöffnungen 46 an den Oberflächenabschnitten 22 und 26 und kleiner als eine Öffnungsweite 80 der Aufnahmeöffnung 46 am Oberflächenabschnitt 24.

Bei der Verbindungseinrichtung 12 bestehen infolge der Durchbrechungen 68, 72 folgende Vorteile:
Durch die Durchbrechung 68 hindurch kann ausgehend vom zweiten Oberflächenabschnitt 24 das Stabelement 16 in die Aufnahmeöffnung 46 am ersten Oberflächenabschnitt 22 eingeführt werden. Ein Pfeil 82 kennzeichnet die diesbezügliche Einführrichtung. Die Einführrichtung 82 ist quer zur Achse 48 und quer zur Fixierrichtung 62 ausgerichtet.

Dadurch besteht die Möglichkeit, das Stabelement 16 nicht nur entlang der Achse 48 in die Aufnahmeöffnung 46 einzuführen, sondern auch über die seitliche Durchbrechung 68. Dies erhöht die Vielseitigkeit der Verbindungseinrichtung 12 und verbessert deren Handhabung. Insbesondere besteht die Möglichkeit, das Stabelement 16 bei bereits vormontiertem Schraubelement 58 in die Aufnahmeöffnung 46 einzuführen.

Vorteilhaft ist es, dass die Aufnahmeöffnung 46 am Oberflächenabschnitt 22 bezüglich der Durchbrechung 68 eine Vertiefung 84 aufweist. Einmal in die Aufnahmeöffnung 46 eingeführt, ist dadurch die Wahrscheinlichkeit verringert, dass das Stabelement 16 unbeabsichtigterweise wieder durch die Durchbrechung 68 hinausgelangt.

Das Stabelement 16 kann beispielsweise durch die Durchbrechung 68 in die Vertiefung 84 eingelegt und darin mittels des Schraubelementes 58 gesichert werden.

Wie insbesondere aus Figur 3 hervorgeht, weist der Grundkörper 18 in Draufsicht längs der Achse 48 am Anschlussbereich 38 im Wesentlichen eine C-Form auf. Im Bereich eines ersten Schenkels, in der Zeichnung oben dargestellt, ist das Fixierelement 20 angeordnet (in der Zeichnungsebene hinter dem Schenkel). An einem zweiten Schenkel, der dem ersten Schenkel gegenüberliegt und in der Zeichnung unten dargestellt ist, ist die Vertiefung 84 angeordnet.

Der zweite Schenkel ist vorliegend kürzer als der erste Schenkel, so dass das freie Ende des zweiten Schenkels in Bezug auf das freie Ende des ersten Schenkels, an dem stirnseitig der Oberflächenabschnitt 24 angeordnet ist, zurückversetzt ist, vorliegend in Richtung des Anschlussbereiches 36.

Die am Beispiel der Aufnahmeöffnung 46 am Oberflächenabschnitt 22 erläuterten Vorteile gelten in entsprechender Weise für die Aufnahmeöffnungen 46 am dritten Oberflächenabschnitt 26 und am zweiten Oberflächenabschnitt 24.

Insbesondere können obige Ausführungen für die Aufnahmeöffnung 46 am dritten Oberflächenabschnitt 26 aufgrund der symmetrischen Ausgestaltung des Grundkörpers 18 unmittelbar übertragen werden. Es besteht die Möglichkeit, das Stabelement 16 durch die weitere Durchbrechung 72 in der Einführrichtung 82 in die Aufnahmeöffnung 46 einzuführen, ausgehend vom zweiten Oberflächenabschnitt 24.

Wie vorstehend am Beispiel des ersten Anschlussbereiches 36 erläutert, kann zum Fixieren des Stabelementes 16 am zweiten Anschlussbereich 38 vorgesehen sein, dass dieses nur durch eine der Aufnahmeöffnungen 46 in den Oberflächenabschnitten 22 oder 26 in den Hohlraum 42 hineinragt und darin mittels des Schraubelementes 58 fixiert wird.

Darüber hinaus besteht die Möglichkeit, dass das Stabelement 16 durch beide Aufnahmeöffnungen 46 an den Oberflächenabschnitten 22 und 26 hindurchragt (Figuren 1 und 6). Hierfür bietet das Vorsehen der Durchbrechungen 68, 72 die Möglichkeit, das Stabelement 16 ausgehend vom zweiten Oberflächenabschnitt 24 in diese beiden Aufnahmeöffnungen 46 in der Einführrichtung 82 einzuführen.

Bei der Aufnahmeöffnung 46 am zweiten Oberflächenabschnitt 24 ist die Möglichkeit gegeben, das Stabelement 16 entlang der Achse 52 einzuführen. Insbesondere kann die Zwischenwand 44 als Anschlagelement 64 wirksam sein.

Durch die Durchbrechung 68 hindurch kann das Stabelement 16 alternativ ausgehend vom ersten Oberflächenabschnitt 22 in die Aufnahmeöffnung 46 am zweiten Oberflächenabschnitt 24 eingeführt werden, quer zur Achse 52 und quer zur Fixierrichtung 62. Ein Pfeil 86 kennzeichnet die diesbezügliche Einführrichtung.

In entsprechender Weise kann das Stabelement 16 alternativ ausgehend vom dritten Oberflächenabschnitt 26 in einer mittels eines Pfeiles 88 gekennzeichneten Einführrichtung in die Aufnahmeöffnung 46 am zweiten Oberflächenabschnitt 24 eingeführt werden, quer zur Achse 52 und quer zur Fixierrichtung 62.

In beiden Fällen kann das Stabelement 16 nach Einführung durch die Durchbrechung 68 oder 72 in die Vertiefung 84 der Aufnahmeöffnung 46 am zweiten Oberflächenabschnitt 24 eingelegt und darin mittels des Schraubelementes 58 fixiert werden.

In den beiden zuletzt genannten Fällen besteht die Möglichkeit einer axialen Verschiebung des Stabelementes 16 solange, bis dieses am Anschlagelement 64 anschlägt.

Bei dem Verbindungssystem 10 besteht der Vorteil, dass das Schraubelement 58 in der Schraubaufnahme 60 am Anschlussbereich 38 vorfixiert sein kann, bevor das Stabelement 16 eingeführt wird. Das Stabelement kann durch eine der Aufnahmeöffnungen 46 an den ersten, zweiten oder dritten Oberflächenabschnitten 22, 24, 26 eingeführt und mittels des bereits vorfixierten Schraubelementes 58 am Anschlussbereich 38 gesichert werden.

Die Aufnahmeöffnungen 46 der Oberflächenabschnitte 24 und 28 sind koaxial zueinander ausgerichtet, um eine axiale Verbindung der Stabelemente 14, 16 zu ermöglichen (Figur 5). Abhängig von den Durchmessern der Stabelemente 14, 16 fluchten deren Achsen beispielsweise miteinander (bei übereinstimmendem Durchmesser), oder deren Achsen sind parallel zu einander ausgerichtet (bei unterschiedlichen Durchmessern). Im letzteren Fall sind die Stabelemente 14, 16 parallel zueinander ausgerichtet.

Eine parallele Relativorientierung der Stabelemente 14, 16 ist dadurch möglich, dass diese in die Aufnahmeöffnungen 46 des ersten und des dritten Oberflächenabschnittes 22, 26 eingeführt werden (Figur 6).

Eine L-förmige Relativorientierung der Stabelemente 14, 16 kann zum einen dadurch erzielt werden, dass das Stabelement 14 in die Aufnahmeöffnung 46 am vierten Oberflächenabschnitt 28 eingeführt wird und das Stabelement 16 in zumindest eine der Aufnahmeöffnungen 46 am ersten und dritten Oberflächenabschnitt 22, 26. Zum anderen kann das Stabelement 16 in die Aufnahmeöffnung 46 am zweiten Oberflächenabschnitt 24 eingeführt sein, und das Stabelement 14 in zumindest eine Aufnahmeöffnung 46 am ersten und am dritten Oberflächenabschnitt 22, 26.

Wie bereits erwähnt ist es günstig, wenn Stabelemente 14, 16 unterschiedlichen Durchmessers in eine jeweilige Aufnahmeöffnung 46 einführbar sind.

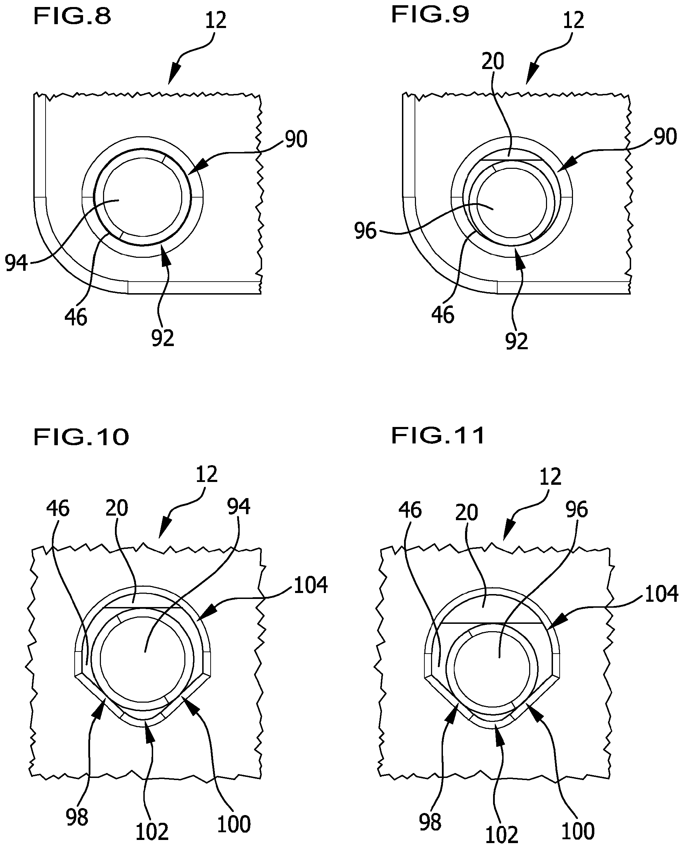

Die Figuren 8 und 9 zeigen eine Detaildarstellung der Aufnahmeöffnung 46 des Anschlussbereiches 36 am Oberflächenabschnitt 22. Zu erkennen ist, dass die Aufnahmeöffnung 46 keine kreisrunde Querschnittsöffnung ist. Stattdessen sind zwei Segmente 90, 92 vorgesehen, die jeweils entlang eines Kreisbogens verlaufen. Allerdings unterscheiden sich die Segmente 90, 92 in ihren Radien. Der Radius des Segmentes 92 ist kleiner als der Radius des Segmentes 90.

Auch die weiteren Aufnahmeöffnungen 46 am Grundkörper 18 weisen Segmente mit unterschiedlichen Radien auf. Die Aufnahmeöffnungen 46 des zweiten Anschlussbereiches 38 umfassen jedoch Segmente 90 unterschiedlich langer Erstreckung, infolge des Vorhandenseins der Durchbrechungen 68, 72.

Das Vorsehen von Segmenten 90, 92 unterschiedlicher Radien bietet den Vorteil, dass Stabelemente unterschiedlichen Durchmessers zuverlässig in der Aufnahmeöffnung 46 fixiert werden können. Figur 8 stellt dies für ein Stabelement 94 mit größerem Radius dar, der an den Radius des Segmentes 90 angepasst ist. Figur 9 zeigt dies für ein Stabelement 96 mit kleinerem Radius, der an den Radius des Segmentes 92 angepasst ist. Es besteht jeweils die Möglichkeit eines flächigen Anliegens am Rand der Aufnahmeöffnung 46.

Die Figuren 10 und 11 zeigen eine andersartige Form einer Aufnahmeöffnung 46 des Grundkörpers 18. Hierbei weist die Aufnahmeöffnung 46 zwei planare und V-förmig zueinander ausgerichtete Segmente 98, 100 auf. Der Winkel zwischen den Segmenten 98, 100 beträgt vorliegend ungefähr 90°.

Zwischen den Segmenten 98, 100 ist ein Segment 102 mit Radius und ungefähr Kreisbogenform vorgesehen. Ein weiteres Segment 104 verbindet die Segmente 98, 100 an ihren voneinander weg weisenden Enden und ist halbkreisförmig mit einem Radius und sich daran anschließend, den Segmenten 98, 100 zugewandt, geradlinig erstreckten Abschnitten versehen.

Anhand der in den Figuren 10 und 11 dargestellten Stabelemente 94 mit größerem Radius und 96 mit kleinerem Radius ist erkennbar, dass eine derartige Ausgestaltung ebenfalls die Möglichkeit einer zuverlässigen Klemmung von Stabelementen unterschiedlichen Durchmessers ermöglicht.

### Bezugszeichenliste

10 Verbindungssystem
12 Verbindungseinrichtung
14, 16 Stabelement
18 Grundkörper
20 Fixierelement
22, 24, 26, 28, 30, 32 Oberflächenabschnitt
34 Mittellängsebene
36, 38 Anschlussbereich
40 Querebene
42 Hohlraum
44 Zwischenwand
46 Aufnahmeöffnung
48, 50, 52, 54 Achse
58 Schraubelement
60 Schraubaufnahme
62 Fixierrichtung
64 Anschlagelement
66, 70, 74 Aufnahmeöffnungsrand
68, 72 Durchbrechung
76, 78, 80 Öffnungsweite
82, 86, 88 Einführrichtung
84 Vertiefung
90, 92, 98, 100, 102, 104 Segment
94, 96 Stabelement

## Patentansprüche

1. Chirurgische Verbindungseinrichtung zum Verbinden eines ersten Stabelementes (14) mit einem zweiten Stabelement (16) in einer oder mehreren, insbesondere zwei, Relativorientierungen, wobei die Verbindungseinrichtung (12) einen Grundkörper (18) umfasst mit einem ersten Anschlussbereich (36) für das erste Stabelement (14) und einem zweiten Anschlussbereich (38) für das zweite Stabelement (16), wobei am Grundkörper (18) mindestens drei Aufnahmeöffnungen (46) gebildet sind, wobei an jedem Anschlussbereich (36, 38) mindestens eine der Aufnahmeöffnungen (46) angeordnet ist und mindestens ein Anschlussbereich (36, 38) zwei oder mehr der Aufnahmeöffnungen (46) mit voneinander unterschiedlichen Achsen (48, 50, 52, 54) aufweist, in welche Aufnahmeöffnungen (46) das Stabelement (14, 16) wahlweise einsetzbar ist, und wobei an jedem Anschlussbereich (36, 38) ein Fixierelement (20) zum Fixieren, in einer Fixierrichtung (62), des jeweiligen Stabelementes (14, 16) am Anschlussbereich (36, 38) angeordnet ist,
und wobei eine der Aufnahmeöffnungen (46) an einem ersten Oberflächenabschnitt (22) des Grundkörpers (18) gebildet ist, deren Aufnahmeöffnungsrand (66) eine Durchbrechung (68) aufweist, die sich vom ersten Oberflächenabschnitt (22) bis zu einem benachbarten, im Winkel ausgerichteten zweiten Oberflächenabschnitt (24) des Grundkörpers (18) erstreckt, zum Einführen des Stabelementes (14, 16) in die Aufnahmeöffnung (46) ausgehend vom zweiten Oberflächenabschnitt (24) und durch die Durchbrechung (68) hindurch, in einer Einführrichtung (82) quer zur Achse (48) der Aufnahmeöffnung (46) und quer zur Fixierrichtung (62),
**dadurch gekennzeichnet, dass** die Aufnahmeöffnung (46) an dem ersten Oberflächenabschnitt (22) bezüglich der Durchbrechung (68) eine Vertiefung (84) bildet oder umfasst, in die das Stabelement (14, 16) einführbar und darin mittels des Fixierelementes (20) fixierbar ist,
dass am zweiten Oberflächenabschnitt (24) eine zweite Aufnahmeöffnung (46) gebildet ist mit einem Aufnahmeöffnungsrand (70), der die Durchbrechung (68) aufweist, zum Einführen des Stabelementes (14, 16) in die zweite Aufnahmeöffnung (46) ausgehend vom ersten Oberflächenabschnitt (22) und durch die Durchbrechung (68) hindurch, quer zur Achse (52) der zweiten Aufnahmeöffnung (46), und
dass die zweite Aufnahmeöffnung (46) bezüglich der Durchbrechung (68) eine Vertiefung (84) bildet oder umfasst, in die das Stabelement (14, 16) einführbar und darin mittels des Fixierelementes (20) fixierbar ist.

2. Verbindungseinrichtung nach Anspruch 1, **dadurch gekennzeichnet, dass** das Fixierelement (20) am Grundkörper (18) vorfixierbar ist, woraufhin das Stabelement (14, 16) durch die Durchbrechung (68) hindurch in die Aufnahmeöffnung (46) einführbar und anschließend mit dem Fixierelement (20) fixierbar ist.

3. Verbindungseinrichtung nach Anspruch 1 oder 2, **dadurch gekennzeichnet, dass** eine Öffnungsweite (76) der Durchbrechung (68) kleiner ist als die Öffnungsweite (78) der Aufnahmeöffnung (46) am ersten Oberflächenabschnitt (22).

4. Verbindungseinrichtung nach einem der voranstehenden Ansprüche , **dadurch gekennzeichnet, dass** der Aufnahmeöffnungsrand (70) der zweiten Aufnahmeöffnung (46) eine weitere Durchbrechung (72) aufweist, zum Einführen des Stabelementes (14, 16) in die zweite Aufnahmeöffnung (46) ausgehend von einem dritten Oberflächenabschnitt (26) des Grundkörpers (18), der dem ersten Oberflächenabschnitt (22) abgewandt ist, und durch die weitere Durchbrechung (72) hindurch, quer zur Achse (52) der zweiten Aufnahmeöffnung (46).

5. Verbindungseinrichtung nach einem der voranstehenden Ansprüche, **dadurch gekennzeichnet, dass** an einem dritten Oberflächenabschnitt (26), der dem ersten Oberflächenabschnitt (22) abgewandt ist, eine dritte Aufnahmeöffnung (46) gebildet ist.

6. Verbindungseinrichtung nach Anspruch 5 in Verbindung mit Anspruch 4, **dadurch gekennzeichnet, dass** ein Aufnahmeöffnungsrand (74) der dritten Aufnahmeöffnung (46) die weitere Durchbrechung (72) aufweist, zum Einführen des Stabelementes (14, 16) in die dritte Aufnahmeöffnung (46) ausgehend vom zweiten Oberflächenabschnitt (24) und durch die weitere Durchbrechung (72) hindurch, quer zur Achse (50).

7. Verbindungseinrichtung nach einem der Ansprüche 1 bis 6, **dadurch gekennzeichnet, dass** eine Öffnungsweite (76) der Durchbrechung (68) kleiner ist als die Öffnungsweite (78) der zweiten Aufnahmeöffnung (46).

8. Verbindungseinrichtung nach einem der Ansprüche 5 bis 7, **dadurch gekennzeichnet, dass** eine Öffnungsweite (76) der weiteren Durchbrechung (72) kleiner ist als eine Öffnungsweite (78) der dritten Aufnahmeöffnung (46) und/oder dass die dritte Aufnahmeöffnung (46) bezüglich der weiteren Durchbrechung (72) eine Vertiefung (84) bildet oder umfasst, in die das Stabelement (14, 16) einführbar und darin mittels des Fixierelementes (20) fixierbar ist.

9. Verbindungseinrichtung nach einem der voranstehenden Ansprüche, **dadurch gekennzeichnet, dass** am ersten Anschlussbereich (36) und am zweiten Anschlussbereich (38) zwei an einander abgewandten Oberflächenabschnitten (24, 28) gebildete Aufnahmeöffnungen (46) gebildet sind, und dass die Stabelemente (14, 16) in einander entgegengesetzten Richtungen in die Aufnahmeöffnungen (46) einführbar sind.

10. Verbindungseinrichtung nach Anspruch 9, **dadurch gekennzeichnet, dass** der Grundkörper (18) ein in einer der Einführrichtungen wirksames Anschlagelement (64) für mindestens eines der Stabelemente (14, 16) umfasst, vorzugsweise dass das Anschlagelement (64) als Zwischenwand (44) des Grundkörpers (18) ausgebildet ist.

11. Verbindungseinrichtung nach einem der voranstehenden Ansprüche, **dadurch gekennzeichnet, dass** zumindest eines der Folgenden gilt:
- am ersten Anschlussbereich (36) ist mindestens eine Aufnahmeöffnung (46) gebildet ist und am zweiten Anschlussbereich (38) mindestens eine weitere Aufnahmeöffnung (46), die Achsen (48, 50, 52, 54) definieren, die quer und insbesondere senkrecht zueinander ausgerichtet sind;
- am ersten Anschlussbereich (36) ist mindestens eine Aufnahmeöffnung (46) gebildet ist und am zweiten Anschlussbereich (38) mindestens eine weitere Aufnahmeöffnung (46), die Achsen (48, 50, 52, 54) definieren, die parallel zueinander ausgerichtet sind;
- an zumindest einem der Anschlussbereiche (36, 38) sind zwei koaxial zueinander ausgerichtete Aufnahmeöffnungen (46) gebildet, wobei das Stabelement (14, 16) durch die beiden Aufnahmeöffnungen (46) hindurch führbar ist.

12. Verbindungseinrichtung nach einem der voranstehenden Ansprüche, **dadurch gekennzeichnet, dass** zumindest eine Aufnahmeöffnung (46) mit einem kreisrunden oder mit einem unrunden Querschnitt vorgesehen ist.

13. Verbindungseinrichtung nach einem der voranstehenden Ansprüche, **dadurch gekennzeichnet, dass** zumindest eine Aufnahmeöffnung (46) vorgesehen ist, deren Aufnahmeöffnungsrand (66, 70, 74) segmentweise entlang eines Kreisbogens mit einem ersten Radius und segmentweise entlang eines Kreisbogens mit einem zweiten Radius verläuft, der zum ersten Radius unterschiedlich ist und insbesondere kleiner als der erste Radius ist und/oder dass zumindest eine Aufnahmeöffnung (46) vorgesehen ist, deren Aufnahmeöffnungsrand (66, 70, 74) zwei planare und V-förmig zueinander ausgerichtete Segmente (98, 100) aufweist.

14. Chirurgisches Verbindungssystem (10), umfassend mindestens eine Verbindungseinrichtung (12) nach einem der voranstehenden Ansprüche und zwei oder mehr Stabelemente (14, 16), die wahlweise am ersten Anschlussbereich (36) und/oder am zweiten Anschlussbereich (38) fixierbar sind.

## Claims

1. A surgical connection device for connecting a first rod element (14) to a second rod element (16) in one or more relative orientations, in particular two, wherein the connection device (12) comprises a main body (18) with a first connection region (36) for the first rod element (14) and a second connection region (38) for the second rod element (16), wherein at least three receiving openings (46) are formed in the main body (18), wherein at least one receiving opening (46) is arranged in each connection region (36, 38) and at least one connection region (36, 38) has two or more receiving openings (46) with different axes (48, 50, 52, 54) from one another, into which receiving openings (46) the rod element (14, 16) is insertable selectively, and wherein in each connection region (36, 38) there is arranged a fixing element (20) for fixing the respective rod element (14, 16) at the connection region (36, 38) in a fixing direction (62),
and wherein one of the receiving openings (46) is formed in a first surface portion (22) of the main body (18), the receiving opening edge (66) of which receiving opening has an aperture (68), which extends from the first surface portion (22) to an adjacent, angled second surface portion (24) of the main body (18), for inserting the rod element (14, 16) into the receiving opening (46) starting from the second surface portion (24) and through the aperture (68), in an insertion direction (82) transverse to the axis (48) of the receiving opening (46) and transverse to the fixing direction (62),
**characterized in that** the receiving opening (46) in the first surface portion (22) forms or comprises a recess (84) with respect to the aperture (68), into which recess the rod element (14, 16) is insertable and therein fixable by means of the fixing element (20),
**in that** in the second surface portion (24) there is formed a second receiving opening (46) with a receiving opening edge (70) which has the aperture (68) for inserting the rod element (14, 16) into the second receiving opening (46) starting from the first surface portion (22) and through the aperture (68), transversely to the axis (52) of the second receiving opening (46), and
**in that** the second receiving opening (46) forms or comprises a recess (84) with respect to the aperture (68), into which recess the rod element (14, 16) is insertable and therein fixable by means of the fixing element (20).

2. A connection device in accordance with claim 1, **characterized in that** the fixing element (20) is prefixable to the main body (18), whereby the rod element (14, 16) is insertable through the aperture (68) into the receiving opening (46) and subsequently fixable with the fixing element (20).

3. A connection device in accordance with claim 1 or 2, **characterized in that** an opening width (76) of the aperture (68) is smaller than the opening width (78) of the receiving opening (46) in the first surface portion (22).

4. A connection device in accordance with the preceding claims , **characterized in that** the receiving opening edge (70) of the second receiving opening (46) has a further aperture (72) for inserting the rod element (14, 16) into the second receiving opening (46) starting from a third surface portion (26) of the main body (18) facing away from the first surface portion (22) and through the further aperture (72), transversely to the axis (52) of the second receiving opening (46).

5. A connection device in accordance with the preceding claims, **characterized in that** a third receiving opening (46) is formed in a third surface portion (26) facing away from the first surface portion (22).

6. A connection device in accordance with claim 5 in conjunction with claim 4, **characterized in that** a receiving opening edge (74) of the third receiving opening (46) has the further aperture (72) for inserting the rod element (14, 16) into the third receiving opening (46) starting from the second surface portion (24) and through the further aperture (72), transversely to the axis (50).

7. A connection device in accordance with claims 1 to 6, **characterized in that** an opening width (76) of the aperture (68) is smaller than the opening width (78) of the second receiving opening (46).

8. A connection device in accordance with claims 5 to 7, **characterized in that** an opening width (76) of the further aperture (72) is smaller than an opening width (78) of the third receiving opening (46) and/or **in that** the third receiving opening (46) forms or comprises a recess (84) with respect to the further aperture (72), into which recess the rod element (14, 16) is insertable and therein fixable by means of the fixing element (20).

9. A connection device in accordance with the preceding claims, **characterized in that** two receiving openings (46) formed in surface portions (24, 28) facing away from one another are formed in the first connection region (36) and in the second connection region (38), and **in that** the rod elements (14, 16) are insertable into the receiving openings (46) in mutually opposite directions.

10. A connection device in accordance with claim 9, **characterized in that** the main body (18) comprises a stop element (64) for at least one of the rod elements (14, 16), which stop element is effective in one of the insertion directions, preferably **in that** the stop element (64) is configured as a partition wall (44) of the main body (18).

11. A connection device in accordance with the preceding claims, **characterized in that** at least one of the following applies:
• at least one receiving opening (46) is formed in the first connection region (36) and at least one further receiving opening (46) in the second connection region (38), which receiving openings define axes (48, 50, 52, 54) which are directed transversely and in particular perpendicularly to one another,
• at least one receiving opening (46) is formed in the first connection region (36) and at least one further receiving opening (46) in the second connection region (38), which receiving openings define axes (48, 50, 52, 54) which are parallel to one another;
• two receiving openings (46) coaxial with one another are formed in at least one of the connection regions (36, 38), and wherein the rod element (14, 16) is guidable through the two receiving openings (46).

12. A connection device in accordance with the preceding claims, **characterized in that** at least one receiving opening (46) is provided with a circular or with a non-round cross section.

13. A connection device in accordance with the preceding claims, **characterized in that** there is provided at least one receiving opening (46) of which the receiving opening edge (66, 70, 74) runs in segments along a circular arc with a first radius and in segments along a circular arc with a second radius, which is different from the first radius and in particular is smaller than the first radius and/or **in that** there is provided at least one receiving opening (46) of which the receiving opening edge (66, 70, 74) has two planar segments (98, 100) oriented in a V shape relative to one another.

14. A surgical connection system (10), comprising at least one connection device (12) in accordance with the preceding claims and two or more rod elements (14, 16) which are fixable selectively to the first connection region (36) and/or to the second connection region (38).

## Revendications

1. Dispositif de connexion chirurgical pour connecter un premier élément de tige (14) à un deuxième élément de tige (16) dans une ou plusieurs orientations relatives, en particulier deux, le dispositif de connexion (12) comprenant un châssis (18) avec une première zone de raccordement (36) pour le premier élément de tige (14) et une deuxième zone de raccordement (38) pour le deuxième élément de tige (16), au moins trois orifices de réception (46) étant formés sur le châssis (18), au moins un des orifices de réception (46) étant disposé sur chaque zone de raccordement (36, 38) et au moins une zone de raccordement (36, 38) présentant deux ou plusieurs orifices de réception (46) avec des axes différents (48, 50, 52, 54), l'élément de tige (14, 16) pouvant être utilisé, au choix, dans les orifices de réception (46), et un élément de fixation (20) étant agencé sur chaque zone de raccordement (36, 38) pour fixer, dans une direction de fixation (62), l'élément de tige respectif (14, 16) sur la zone de raccordement (36, 38) et dans lequel l'un des orifices de réception (46) est formé sur une première section de surface (22) du châssis (18), dont le bord de l'orifice de réception (66) présente une découpe (68) qui s'étend de manière inclinée de la première section de surface (22) à une deuxième section de surface (24) adjacente au châssis (18) pour insérer l'élément de tige (14, 16) dans l'orifice de réception (46) à partir de la deuxième section de surface (24) et à travers la découpe (68), dans une direction d'insertion (82) transversale à l'axe (48) de l'orifice de réception (46) et transversale à la direction de fixation (62),
**caractérisé en ce que** l'orifice de réception (46) sur la première section de surface (22) forme ou comprend un évidement (84) par rapport à la découpe (68), dans lequel l'élément de tige (14, 16) peut être inséré et fixé au moyen de l'élément de fixation (20),
**en ce qu'**un deuxième orifice de réception (46) est formé sur la deuxième section de surface (24) avec un bord d'orifice de réception (70) qui présente la découpe (68) pour insérer l'élément de tige (14, 16) dans le deuxième orifice de réception (46) en partant de la première section de surface (22) et à travers la découpe (68), transversalement à l'axe (52) du deuxième orifice de réception (46), et
**en ce que** le deuxième orifice de réception (46) forme ou comprend un évidement (84) par rapport à la découpe (68), dans lequel l'élément de tige (14, 16) peut être inséré et fixé au moyen de l'élément de fixation (20).

2. Dispositif de connexion selon la revendication 1, **caractérisé en ce que** l'élément de fixation (20) peut être fixé au préalable au châssis (18), après quoi l'élément de tige (14, 16) peut être inséré à travers la découpe (68) dans l'orifice de réception (46) et peut ensuite être fixé avec l'élément de fixation (20).

3. Dispositif de connexion selon la revendication 1 ou 2, **caractérisé en ce qu'**une largeur d'ouverture (76) de la découpe (68) est inférieure à la largeur d'ouverture (78) de l'orifice de réception (46) sur la première section de surface (22).

4. Dispositif de connexion selon l'une des revendications précédentes, **caractérisé en ce que** le bord de l'orifice de réception (70) du deuxième orifice de réception (46) présente une autre découpe (72) pour insérer l'élément de tige (14, 16) dans le deuxième orifice de réception (46) à partir d'une troisième section de surface (26) du châssis (18) opposée à la première section de surface (22) et traverse l'autre découpe (72), transversalement à l'axe (52) du deuxième orifice de réception (46).

5. Dispositif de connexion selon l'une des revendications précédentes, **caractérisé en ce qu'**un troisième orifice de réception (46) est formé sur une troisième section de surface (26) opposée à la première section de surface (22).

6. Dispositif de connexion selon la revendication 5 prise conjointement avec la revendication 4, **caractérisé en ce qu'**un bord d'orifice de réception (74) du troisième orifice de réception (46) présente l'autre découpe (72) pour insérer l'élément de tige (14, 16) dans le troisième orifice de réception (46) à partir de la deuxième section de surface (24) et à travers l'autre découpe (72), transversalement à l'axe (50).

7. Dispositif de connexion selon l'une des revendications 1 à 6, **caractérisé en ce qu'**une largeur d'ouverture (76) de la découpe (68) est inférieure à la largeur d'ouverture (78) du deuxième orifice de réception (46).

8. Dispositif de connexion selon l'une des revendications 5 à 7, **caractérisé en ce qu'**une largeur d'ouverture (76) de l'autre découpe (72) est inférieure à une largeur d'ouverture (78) du troisième orifice de réception (46) et/ou **en ce que** le troisième orifice de réception (46) forme ou comprend un évidement (84) par rapport à la découpe supplémentaire (72) dans laquelle l'élément de tige (14, 16) peut être inséré et fixé au moyen de l'élément de fixation (20).

9. Dispositif de connexion selon l'une des revendications précédentes, **caractérisé en ce que** deux orifices de réception (46) formés sur des sections de surface (24, 28) opposées l'une à l'autre sont formés sur la première zone de raccordement (36) et sur la deuxième zone de raccordement (38), et **en ce que** les éléments de tige (14, 16) peuvent être insérés dans les orifices de réception (46) dans des directions opposées.

10. Dispositif de connexion selon la revendication 9, **caractérisé en ce que** le châssis (18) comprend un élément de butée (64) qui agit dans l'une des directions d'insertion pour au moins un des éléments de tige (14, 16) et **en ce que**, de préférence, l'élément de butée (64) sert à former une cloison de séparation (44) du châssis (18).

11. Dispositif de connexion selon l'une des revendications précédentes, **caractérisé en ce qu'**au moins l'un des critères suivants s'applique:
- au moins un orifice de réception (46) est formé sur la première zone de raccordement (36) et au moins un deuxième orifice de réception (46) est formé sur la deuxième zone de de raccordement (38), qui définissent des axes (48, 50, 52, 54) alignés transversalement et, en particulier, perpendiculairement l'un à l'autre ;
- au moins un orifice de réception (46) est formé sur la première zone de raccordement (36) et au moins un deuxième orifice de réception (46) est formé sur la deuxième zone de raccordement (38), qui définissent des axes (48, 50, 52, 54) qui sont alignés parallèlement les uns aux autres ;
- deux orifices de réception (46) alignés coaxialement l'un par rapport à l'autre sont formés sur au moins une des zones de raccordement (36, 38), l'élément de tige (14, 16) pouvant être guidé à travers les deux orifices de réception (46).

12. Dispositif de connexion selon l'une des revendications précédentes, **caractérisé en ce qu'**au moins un orifice de réception (46) est prévu avec une section transversale circulaire ou non circulaire.

13. Dispositif de connexion selon l'une des revendications précédentes, **caractérisé en ce qu'**il est prévu au moins un orifice de réception (46) dont le bord d'orifice de réception (66, 70, 74) s'étend par segments le long d'un arc de cercle d'un premier rayon et par segments le long d'un arc de cercle d'un deuxième rayon qui est différent du premier rayon et, en particulier, est plus petit que le premier rayon et/ou **en ce qu'**au moins un orifice de réception (46) est prévu dont le bord de l'orifice de réception (66, 70, 74) comporte deux segments plans et en forme de V (98, 100) alignés l'un avec l'autre.

14. Système de connexion chirurgical (10) comprenant au moins un dispositif de connexion (12) selon l'une des revendications précédentes et deux ou plusieurs éléments de tige (14, 16) qui peuvent être fixés, au choix, sur la première zone de raccordement (36) et/ ou sur la deuxième zone de raccordement (38).
